(19) Europäisches Patentamt
European Patent Office
Office européen des brevets

(11) EP 1 927 664 A1

(12) EUROPEAN PATENT APPLICATION

(43) Date of publication:
04.06.2008 Bulletin 2008/23

(51) Int Cl.:
*C12Q 1/68* (2006.01)

(21) Application number: 07022954.7

(22) Date of filing: 27.11.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(30) Priority: 28.11.2006 JP 2006319976

(71) Applicant: CANON KABUSHIKI KAISHA
Ohta-ku, Tokyo (JP)

(72) Inventors:
• Suzuki, Tomohiro
Tokyo (JP)

• Kato, Ayako
Tokyo (JP)

(74) Representative: Weser, Thilo
Weser & Kollegen
Patentanwälte
Radeckestrasse 43
81245 München (DE)

Remarks:
A request for correction to the description has been
filed pursuant to Rule 139 EPC. A decision on the
request will be taken during the proceedings before
the Examining Division (Guidelines for Examination
in the EPO, A-V, 3.).

(54) **Primer for bacterium genome amplification reaction**

(57)     A primer or a primer set can efficiently amplify the gene sequence of 16S rRNA of a target bacterium out of selected sixty two bacterium species. At least one of the primers having a base sequence selected from sequences No. 1 through No. 6 is employed as bacterium genome amplification reaction primer:

(1)     GCGGCGTGCCTAATACATGCAAGTCG (sequence No. 1)

(2)     GCGGCAGGCCTAACACATGCAAGTCG (sequence No. 2)

(3)     GCGGCAGGCTTAACACATGCAAGTCG (sequence No. 3)

(4) ATCCAGCCGCACCTTCCGATACGGC (sequence No. 4)

(5)     ATCCAACCGCAGGTTCCCCTACGG (sequence No. 5)

(6) ATCCAGCCGCAGGTTCCCCTACGG (sequence No. 6).

EP 1 927 664 A1

**Description**

BACKGROUND OF THE INVENTION

Field of the Invention

The present invention relates to a primer to be used for genome amplification reaction in the 16S rRNA code region of a bacterium. The present invention also relates to a primer set of a combination of such primers, a kit having such a primer set and a method of detecting bacteria by means of a primer set.

Description of the Related Art

Conventional culture processes have been and being mainly employed to identify the causative bacteria that cause the infectious diseases in objects of examination such as blood and sputum. The causative bacterium is then identified by seeing the profiles of colonies and the growth condition of each culture medium. Proposals have been made to improve the existing techniques of identifying the causative bacterium and provide new techniques in order to make the treatment and the medication for the patient faster and more appropriate.

Typical newly proposed techniques include those of analyzing the DNA sequence of a bacterium and identifying the species of the bacterium from the DNA sequence. For example, Ezaki et al. propose a bacterium identification method using a DNA chip where chromosome DNAs are anchored as DNA probe in Japanese Patent Application Laid-Open Publication No. 2001-299396. With the proposed method, it is possible to detect the unknown bacterium in an object of examination by making the chromosome DNAs originating from a plurality of known bacteria having respective GC contents that are different from each other react with the chromosome DNA originating from the unknown bacterium in the object of examination and detecting the produced hybridization complexes.

Ohno et al. propose a fungus detection probe utilizing a restriction enzyme fragment as probe to be used in DNA chip for detecting the infective bacteria in an infectious disease in Japanese Patent Application Laid-Open No. H06-133798. Ohno et al. further proposes a Pseudomonas aeruginosa detection probe and a detection probe utilizing restriction enzyme fragments of *Escherichia coli, Klebsiella pneumoniae* and *Enterobacter cloacae* respectively in Japanese Patent Application Laid-Open No. H10-394896 and in Japanese Patent Application Laid-Open No. H10-304897.

Japanese Patent Application Laid-Open No. 2004-313181 discloses a DNA chip prepared by using oligonucleotides having a relatively short chain length as probes in order to raise the detection accuracy. The disclosed chip can identify bacteria of the ten different species as listed below that are detected clinically highly frequently as infective bacteria.

*S. aureus, S. epidermidis, E. coli*, *K. pneumoniae, P. aeruginosa, S. marcescens, S. pneumoniae, H. influenzae, E. cloacae* and *E. faecalis.*

With the method disclosed in the above-cited Patent Document, bacterium-specific oligo probes are selected for those bacterium species to see the bacterium of which probe hybridizes with the DNA in the object of examination and identify the bacterium species. More specifically, the DNA in the object of examination is extracted and subsequently amplified by PCR in a preliminary process in order to amplify and label the target nucleic acid region. Then, the obtained PCR product is made to hybridize with any of the probes of the DNA chip and the bacterium species is identified on the basis of the luminance data of the DNA chip.

The method of identifying the type of bacterium of Japanese Patent Application Laid-Open Publication No. 2004-313181 involves a preliminary process of PCR amplification. While PCR amplification is a highly efficient technique for amplifying nucleic acids, the significance thereof varies depending on the quantity of the template contained in the object of examination. For example, the number of bacteria cultured on an agar medium is very large and hence it is possible to achieve a sufficient degree of detection sensitivity when a PCR provides an amplification effect of a certain level. In case that the object of examination is human blood, a sufficient amount of bacterium genome, or a sufficient amount of template can be secured if the specimen is cultured over a full day, and grown to a sufficient level of amplification.

However, a very high level of detection sensitivity is required when the bacterium contained in human blood is not cultured (amplified) and the bacterium species is identified directly because the bacterium concentration in blood is very low and 1 mL of blood contains tens to hundreds of living bacteria at most in the case of sepsis that shows a high bacterium concentration. When the bacteria in blood are not condensed, the quantity that can be brought in as template for PCR amplification is about 0.3 mL at most as reduced to blood. Then, the quantity of genome originating from the bacterium is maximally equal to that of the genomes of about 100 bacteria. As a matter of course, the bacterium concentration in blood is lower in the case of a less serious infectious disease.

With PCR amplification, the quantity of template is theoretically doubled in each cycle and hence it is possible to start with a single copy of template for PCR amplification. However, the efficiency of PCR amplification is generally not higher than twice and it is not rare that the template is apparently practically not amplified when the quantity of template is small. There are various factors that raise or lower the PCR efficiency and they typically include the type of enzyme, the thermal cycle program, the primer sequence and the buffer conditions, of which the primer sequence is important for raising or lowering the amplification efficiency. Therefore, it is important to select a right primer.

SUMMARY OF THE INVENTION

In order to enable to efficiently amplify bacteria of a number of different species (e.g., 62 species), it is necessary to prepare a mixture of primers of two or more than two different kinds. However, it is often difficult to design each primer and select an appropriate combination of primers.

Thus, it is the object of the present invention to provide a primer or a primer set that can efficiently amplify the 16S rRNA gene sequence of the target bacterium selected from a large number of species of bacterium, sixty-two species in particular.

As a result of intensive research efforts, the inventor of the present invention came to find that a primer having the base sequence selected from the sequences No. 1 through No. 6 listed below is effective as primer for amplifying a specific region of 16S rRNA selected from sixty-two bacterium species. Thus, the present invention in its first aspect provides a primer as specified in claim 1.

The present invention in its second aspect provides a primer set as specified in claims 2 to 4 and 7.

The present invention in its third and fourth aspects provides a reagent kit as specified in claim 5 and a bacterium detection method as specified in claim 6, respectively.

Thus, very efficient PCR amplification of a specific region of 16S rRNA of any of the sixty-two bacterium species has become possible by a primer according to the present invention. It has also become possible to very efficiently amplify bacteria of all the sixty two species by means of a primer or a primer set formed by appropriately combining primers according to the present invention.

Further features of the present invention will become apparent from the following description of exemplary embodiments.

DETAILED DESCRIPTION OF THE INVENTION

A primer according to the present invention is a primer for amplifying a specific region of 16S rRNA of the genome of a bacterium. It is a primer having a base sequence selected from the sequences No. 1 through No. 6 listed below.

(1) GCGGCGTGCCTAATACATGCAAGTCG (sequence No. 1)
(2) GCGGCAGGCCTAACACATGCAAGTCG (sequence No. 2)
(3) GCGGCAGGCTTAACACATGCAAGTCG (sequence No. 3)
(4) ATCCAGCCGCACCTTCCGATACGGC (sequence No. 4)
(5) ATCCAACCGCAGGTTCCCCTACGG (sequence No. 5)
(6) ATCCAGCCGCAGGTTCCCCTACGG (sequence No. 6)

A primer according to the present invention can be applied to any bacterium or an object originating from a living thing so long as it has a genome that allows PCR amplification regardless of agreement or disagreement of sequence. A primer according to the present invention can mainly find suitable applications of amplifying the following sixty-two bacterium species.

*S. aureus, S. epidermidis, E. coli, K. pneumoniae, P. aeruginosa, S. marcescens, S. pneumoniae, H. influenzae, E. cloacae, E. faecalis, S. haemolyticus, S. hominis, S. saprophyticus, P. fluorescens, P. putida, B. cepacia, A. baumannii, B. cereus, B. subtilis, A. calcoaceticus, A. xylosoxidans, S. choleraesuis, M. Chelonae, N. asteroids, K. oxytoca, E. aerogenes, H. alvei, S. liquefaciens, P. mirabilis, P. vulgaris, M. morganii, P. rettgeri, A. hydrophila, S. agalactiae, S. mutans, S. pyogenes, S. sanguinis,*

*E. avium, E. faecium, S. maltophilia, C. freundii, A. sobria, V. vulnificus, G. vaginalis, B. fragilis, B. thetaiotaomicron, P. acnes, C. difficile, C. perfrigens, E. lenta, F. nucleatum, L. acidophilus, A. prevotii, P. asaccharolyticus, P. asaccharolytica, P. gingivalis, C. diphtheriae, L. pneumophila, M. Kansasii, M. intracellulare, F. necrophorum and P. denticola.*

A primer according to the present invention is designed so as to be able to specifically recognize the 16S rRNA regions and amplify them by PCR. In other words, the primer is designed not only to show a specific sequence necessary for amplifying the target region of the DNA sequence of a bacterium but also not to show any sequence resembling a DNA sequence originating from a human being that can coexist at the time of collecting bacterium genomes from blood. On the other hand, a sequence to uniformly amplify the target bacteria regardless of the difference of strain in the bacterium species and mutation is adapted.

The target object of examination needs to be in a condition where the PCR reagent can directly react as in the case of the DNA extracted from a bacterium, although the process of getting to that condition is not subjected to any limitation. In other words, any bacteria that are cultured on a liquid culture medium or an agar culture medium can be used so long as their genomes can be extracted or react to the PCR reagent. The object of examination can be selected from any specimens originating from a human being and domestic animals where bacterium can exist including body fluids including bloods, components of bloods, spiral fluids, sputums, gastric juices, vaginal discharges and intraoral mucus and excrements including urine and feces. Additionally, the object of examination can also be all media that can be contaminated by bacteria such as foods and beverages that can be food poisoned or contaminated, waters in the environment such as hot spring waters or filters of air cleaners. Still additionally, the object of examination can also be selected from specimens taken from animals and plants under quarantine scrutiny for export and import. Furthermore, the object of examination can also be selected from objects acquired by any of various biochemical techniques such as various reagents, PCR products and nucleic acid fragments treated by a restriction enzyme.

A primer according to the present invention can be used with any technique relating to PCR amplification that is being generally used. It is possible to select a primer each for the forward side (F chain) and the reverse side (R chain) with an arbitrarily selected concentration and subject them to PCR amplification. It is also possible to use only one of the chains for asymmetric PCR amplification. It is also possible to make a primer according to the present invention taken in an arbitrarily selected label any position thereof. For example, a fluorescent substance or a radiation isotope may be labeled.

A primer according to the present invention can be used in a mixed primer obtained by mixing F chains and R chains of a plurality of different types. The primer mix may be configured arbitrarily according to the object of amplification. A primer set including at least two of the primers (A) through (L) listed below, at least one of which is selected from the primers (A) through (F), may preferably be used.

(A) a primer having GCGGCGTGCCTAATACATGCAAGTCG (sequence No. 1),
(B) a primer having GCGGCAGGCCTAACACATGCAAGTCG (sequence No. 2),
(C) a primer having GCGGCAGGCTTAACACATGCAAGTCG (sequence No. 3),
(D) a primer having ATCCAGCCGCACCTTCCGATACGGC (sequence No. 4),
(E) a primer having ATCCAACCGCAGGTTCCCCTACGG (sequence No. 5),
(F) a primer having ATCCAGCCGCAGGTTCCCCTACGG (sequence No. 6),
(G) a primer having GCGGCGTGCCTAATACATGCAAG (sequence No. 7),
(H) a primer having GCGGCAGGCCTAACACATGCAAG (sequence No. 8),
(I) a primer having GCGGCAGGCTTAACACATGCAAG (sequence No. 9),
(J) a primer having ATCCAGCCGCACCTTCCGATAC (sequence No. 10),
(K) a primer having ATCCAACCGCAGGTTCCCCTAC (sequence No. 11) and
(L) a primer having ATCCAGCCGCAGGTTCCCCTAC (sequence No. 12).

A primer set including as least six primers of (D) through (I) and a primer set including at least six primers of (D), (F), (G), (H), (I) and (K) are preferable.

Furthermore, a primer set prepared by mixing the six primers of (D) through (I) by the same amounts and a primer set prepared by mixing the six primers of (D), (F), (G), (H), (I) and (K) by the same amounts are preferable. Such a primer set can amplify the above listed sixty-two bacterium species in a well balanced manner. If the bacterium species contained in the object of examination is not known, a primer set according to the present invention can suitably be used for PCR amplification in order to analyze the PCR product by some means or another.

When two or more than two primers are used as mixture in a primer set according to the present invention, it is possible to arbitrarily and independently label each of the component primers.

Any enzyme (thermostable DNA polymerase) that can be used for PCR can be used without limitation. Typical enzymes include ExTaq manufactured by Takara Bio Inc., AmpliTaq Gold manufactured by ABI (Applied Biosystems) and AccuPrime manufactured by Invitrogen Corporation. In a PCR amplification reaction, it is possible to mix a labeled substrate with a PCR reaction solution in addition to an ordinary substrate and make the PCR product take in a label originating from the substrate.

A primer and a primer set according to the present invention can be used for various applications without any particular limitation. For example, a primer or a primer set according to the present invention can be used as a probe anchored to the solid phase carrier of a DNA microarray. If such is the case, a primer according to the present invention and showing a high amplification rate can suitably be used to efficiently prepare a DNA microarray. When looking into an object of observation (object of examination) to find out the bacterium species the object of examination contains, a primer according to the present invention can perform amplification very efficiently. While any of various techniques may be used for analyzing the PCR reagent product (amplification product), popular analysis techniques include an electrophoresis method, a sequence method, a quantitative PCR method and an analysis method using a DNA microarray. Of these, the technique of using a DNA microarray where a probe for detecting the target nucleic acid as object of detection is anchored can particularly advantageously be used because the sequence in the amplification region can be analyzed quantitatively in a simpler manner. More specifically, an object of examination where it is not known if any bacterium can be found or not is subjected to PCR amplification by means of a primer set that can amplify all the sixty two bacterium species to obtain a PCR product. The obtained PCR product is labeled by way of re-PCR using a labeling primer. The labeled PCR product is made to hybridize with a DNA microarray and the microarray is observed to see the probe on the microarray that actually worked for hybridization and the extent of hybridization is quantified. An oligo probe showing a characteristic sequence that the target bacterium species has is employed. Such an oligo probe is designed to principally work for hybridization when the target bacterium species is contained in the PCR product.

It is also possible to package a primer or a primer set according to the present invention and provide the package as kit. It is sufficient for the kit to contain at least a primer selected from the primers (A) through (F). In other words, it is possible to provide a kit where at least a primer is incorporated. Such a primer or a primer set can be provided in any form. While such a primer is popularly dissolved in a buffer solution or a liquid medium such as water and supplied as liquid, it is also possible to supply such a primer or a primer set in the form of powder having a liquid component only to a small extent. It is also possible to supply such a primer or a primer set adsorbing thinly on the surface of beads, a glass carrier or resin. When such a primer or a primer set is supplied in the form of a kit, it is more often than not supplied in combination with one or more than one enzymes for PCR, a buffer and/or a labeling substance. In other words, such a primer or a primer set can be combined with any of such substances and supplied as kit. Particularly, when such a primer or a primer set is combined with a DNA microarray and supplied as "kit for identifying bacterium species", the kit can find applications for identifying the bacterium species that causes an infectious disease. Thus, according to present invention, there is provided a method of detecting bacteria by means of a primer, a primer set or a kit according to the present invention.

[Examples]

PCR amplification experiments were conducted on the sixty two bacterium species listed above by using primers according to the present invention. The results of the experiments are described below in detail. Note that the Examples described below do not limit the scope of the present invention at all.

Example 1 (10 bacterium species)

1. Culture of bacteria

Standard strains of bacteria of the ten species that are main objects of detection for the purpose of the present invention and described in ATCC were obtained by following the predetermined procedures. The obtained strains of bacteria were treated for storage in a proper way and then frozen (-80°C) and stocked. The ten species of bacteria are listed below: *S. aureus, S. epidermidis, E. coli, K. pneumoniae, P. aeruginosa, S. marcescens, S. pneumoniae, H. influenzae, E.. cloacae, E. faecalis.*

The surfaces of the frozen stocks of bacteria were scraped off to a small extent by a platinum loop and the bacteria obtained by the scraping were applied onto respective plate culture mediums. A chocolate agar culture medium was used only for *H. influenzae* and sheep blood agar culture mediums were used for the remaining nine bacterium species. The bacteria were cultured at 37°C, while being observed for growth. As a result, all the species were proliferated in about 1 to 2 days. A bacterium solution of an appropriate bacterium concentration was prepared for each species by using a turbidity meter and other tools.

2. Extraction of bacterium genome and human genome

The genome DNA of each of the bacterium species was extracted and purified from the corresponding one of the bacterium culture solutions by means of a nucleic acid purification kit (FastPrep FP100A · FastDNA Kit manufactured by Funakoshi Corporation). The collected genome DNA was subjected to agarose electrophoresis and 260/280 nm absorbance measurement by way of an established process to determine the quality (the mixing ratio of low molecular weight nucleic acids, the extent of decomposition) and the collected quantity thereof. In this example, about 5 to 15 $\mu$g of genome DNA originating from the bacterium were collected for each of the extracted ten bacterium species, although the collected quantity varied from species to species. The quality of the collected genome DNA was excellent and neither degradation nor mixed rRNA was observed. The collected genome DNA was diluted in TE to prepare a solution containing genome DNA of ten bacteria per 1 $\mu$l.

Meanwhile, genome originating from a human being was extracted from the blood drawn from a healthy person. A nucleic acid purification kit (QIAamp DNA Blood Mini Kit manufactured by QIAGEN) was used to extract human genome originating from the blood. About 8 $\mu$g of genome DNA were extracted from about 200 $\mu$l of blood. The obtained genome DNA was dissolved in TE to prepare a solution showing a concentration of 200 ng per 1 $\mu$l. The bacterium genome and the human genome were mixed by the same quantities to obtain a model object of examination (specimen) of a genome mixture solution containing 10 copies of the bacterium genome and 200 ng of the human genome per 2 μl.

3. Preparation of primers

The nucleic acid sequence as shown below is designed as PCR primer for amplifying the 16S rRNA gene (target gene) for the purpose of detecting a bacterium. More specifically, a probe set that specifically amplifies the genome part coding 16S rRNA, wherein primers are corresponding to the both ends of the 16S rRNA coding region of about 1,400 to 1,700 base length, specific melting temperature thereof agree with each other as much as possible was employed. Note that primers of a plurality of different types are designed to enable to amplify mutant strains and the plurality of 16S rRNA genes existing on the genome at the same time.

(A) ATCCAGCCGCACCTTCCGATACGGC (sequence No. 4)
(B) ATCCAGCCGCAGGTTCCCCTACGG (sequence No. 6)
(C) GCGGCGTGCCTAATACATGCAAG (sequence No. 7)
(D) GCGGCAGGCCTAACACATGCAAG (sequence No. 8)
(E) GCGGCAGGCTTAACACATGCAAG (sequence No. 9)
(F) ATCCAACCGCAGGTTCCCCTAC (sequence No. 11)

The primers were synthesized by means of a DNA automatic synthesizer and purified by means of high performance liquid chromatography (HPLC) after the synthesis. Each of the primers was dissolved in a tris-hydrochloric acid EDTA buffer solution (1 × TE) to produce 3.0 μM. Same quantities of the primer solutions of the six different types A to F were mixed with each other to prepare a primer mix (primer set) of the primers of the six types with a content of 0.5 μM.

4. PCR amplification

A PCR was conducted in a manner as described below by using each of the prepared primer sets. An enzyme (kit) of AmpliTaq Gold LD type (manufactured by Applied Biosystems) was used as PCR enzyme kit. The primer sets were prepared according to the annexed instruction of the enzyme kit. A PCR reaction solution having the contents as listed below was prepared for each of the primer sets.

[Table 1]

| | |
|---|---|
| AmpliTaq Gold LD Polymerase (5.0unit/μl) | 0.5 μl (which correspond to 2.5 units) |
| 10 × AmpliTaq Gold Buffer | 5.0 μl |
| MgCl$_2$ (25 mM) | 7.0 μl (3.5 mM) |
| dNTP Mix (2.5 mM each) | 4.0 μl (each corresponds to 0.2 mM) |
| primer mix | 5.0 μl (each corresponds to 0.05 μM) |
| H$_2$O | 26.5 μl |
| Template DNA (prepared in Examples 1-2) | 2.0μl |

A commercially available thermal cycler was used for the PCR. The temperatures of the thermal cycler were set as follows.

| | | |
|---|---|---|
| a) | 95°C | 600 seconds |
| b) | 92°C | 45 seconds |
| c) | 65°C | 45 seconds total of 40 cycles of b) → d) |
| d) | 72°C | 45 seconds |
| e) | 72°C | 600 seconds |
| f) | 4°C | storage |

5. Electrophoresis

Each of the obtained PCR products was subjected to electrophoresis in order to observe the quantity of amplification obtained by the amplification reaction. A micro-chip type apparatus for electrophoresis "Bioanalyzer" manufactured by Agilent was used for the electrophoresis. Each of the PCR products was applied to a chip of the apparatus by 1.0 μl and subjected to electrophoresis according to the instruction annexed to the apparatus. After the electrophoresis, the synthesized quantity of the PCR product was quantified by means of the software annexed to the apparatus. The chain length of the PCR product to be amplified was about 1,500 bp. Table 2 below shows the PCR products of all the ten bacterium species.

[Table 2]

| Bacterium species | Amplified quantity (concentration) : ng/μl |
|---|---|
| S. aureus | 0.99 |
| S. epidermidis | 1.08 |
| E. coli | 2.19 |
| K. pneumoniae | 2.41 |
| P. aeruginosa | 2.96 |
| S. marcescens | 8.44 |
| S. pneumoniae | 1.01 |
| H. influenzae | 1.74 |
| E. cloacae | 1.67 |
| E. faecalis | 1.35 |

All the bands show respective lengths that are close to their theoretical chain lengths and no remarkable band was observed with other chain length so that it was confirmed that the genomes originating from the bacteria had been amplified very efficiently. The extent of amplification did not remarkably vary from bacterium species to bacterium species to prove that the primer mix had operated uniformly to all the ten bacterium species. While 200 ng of a genome originating from a human being was added per PCR tube in this example, the human genome was not amplified, therefore it was proved that a human genome does not inhibit such amplification.

Example 2

1. Preparation of DNA microarray

A DNA microarray that can identify the ten bacterium species was prepared. The DNA microarray prepared in this example was designed so as to enable to identify the bacterium species by analyzing each of the sequence of 16S rRNA amplified in Example 1 in detail. In other words, the DNA microarray prepared in this example is a DNA microarray where oligonucleotide probes specifically reactive to the respective bacterium species were designed and mounted on a glass carrier as minute spots.

The probes designed respectively for the bacterium species are listed below.

Probe A: *S. aureus:* 5' TAACCTTTTAGGAGCTAGCCGTCGA 3' (sequence No. 13)
Probe B: *S. epidermidis:* 5'AGTAACCATTTGGAGCTAGCCGTC 3' (sequence No. 14)
Probe C: *E. coli:* 5'CGGACCTCATAAAGTGCGTCGTAGT 3' (sequence No. 15)
Probe E: *K. pneumoniae*: 5'CCTTTGTTGCCAGCGGTTAGGC 3' (sequence No. 16)
Probe F: *P. aeruginosa:* 5' TGGCCTTGACATGCTGAGAACTTTC 3' (sequence No. 17)
Probe G: *S. marcescens*: 5'GAAACTGGCAAGCTAGAGTCTCGTAGA 3' (sequence No. 18)
Probe H: *S. pneumoniae*: 5' GACGGCAAGCTAATCTCTTAAAGCCA 3' (sequence No. 19)
Probe I: *H. influenzae*: 5'GGCGTTTACCACGGTATGATTCATGA 3' (sequence No. 20)
Probe J: *E. cloacae:* 5' ATTCGAAACTGGCAGGCTAGAGTCT 3' (sequence No. 21)
Probe K: *E. faecalis:* 5' CGAGGTCATGCAAATCTCTTAAAGCTTCT 3' (sequence No. 22)

The DNA microarray mounted with these oligonucleotide probes was prepared according to the process described in Japanese Patent Application Laid-Open No. 2004-313181.

2. 2nd PCR

The PCR products obtained in Example 1 were processed for labeling and other operations. Firstly, the PCR products obtained in Example 1 were purified by way of an established process. The kits used for the purification were QIAquick PCR Purification Kits marketed by QIAGEN. Each of the PCR products was made to be adsorbed to the column supplied in the kit and eluted by means of 50 μl of water. The obtained purified PCR product was then analyzed by means of a bioanalyzer as in Example 1 to determine the DNA concentration of the corresponding bacterium species. Then, primers G were synthesized for the 2nd PCR. Since it was necessary to visualize each of the primers in the subsequent experiments, each of the primers was labeled with Cy3 at the 5' end. After the synthesis, the primers were purified by means of high performance liquid chromatography (HPLC). The primers G were dissolved in a TE buffer solution at the concentration of 10 pmol/μl.

G) TACCTTGTTACGACTTCACCCCA (sequence No. 23)

The primer G1 was subjected to an asymmetric PCR, using the obtained PCR products as templates. The PCR was conducted by way of the following preparation process. While each of the DNAs purified in the first PCR was used by 20 μl in principle for the DNA to be used for the corresponding template, the solution was diluted by water when the total quantity of the DNA could exceed 30 ng so that the total quantity might not exceed 30 ng. (*)

[Table 3]

· PCR mix

| | |
|---|---|
| ExTaq manufactured by Takara Bio Inc, (premixed type 2×) | 25.0 μl |
| primer G | 5.0 μl |
| $H_2O$ | 0 μl (*) |
| Template DNA | 20.0 μl (*) |

The temperatures of the thermal cycler were set as follows.

| a) | 95°C | 600 seconds |
|---|---|---|
| b) | 92°C | 45 seconds |
| c) | 65°C | 45 seconds total of 25 cycles of b) → d) |
| d) | 72°C | 45 seconds |
| e) | 72°C | 600 seconds |
| f) | 4°C | storage |

The obtained PCR products were purified by Purification Kits manufactured by QIAGEN in same way as described above. (eluted by 50 μl of water)

3. Hybridization

The DNA microarray prepared in 1. preparation of DNA microarray above and the labeled object of examination (specimen) prepared in 2. 2nd PCR above were used for a hybridization reaction.

3-1. Blocking of DNA microarray

BSA (bovine serum albumin: Fraction V manufactured by Sigma) was dissolved in 100 mM of NaCl / 10 mM of phosphate buffer to show 1 wt%. The prepared DNA microarray was immersed in the solution at room temperature for 2 hours and subjected to a blocking process. After the blocking, the DNA microarray was washed with washing liquid as shown below and subsequently rinsed with pure water and the DNA microarray was drained by means of a spin drier.

Washing liquid:

2 × SSC solution (NaCl 300 mM, sodium citrate (trisodium citrate dehydrate, $C_6H_5Na_3 \cdot 2H_2O$) 30 mM, pH. 7.0) containing SDS (sodium dodecyl sulfate) by 0.1 wt%.

3-2. Hybridization

The drained DNA chip was set in a hybridization apparatus (Hybridization Station, manufactured by Genomic Solutions Inc.) and subjected to a hybridization reaction in a hybridization solution and under the condition listed below.

3-3. Hybridization solution

```
6 × SSPE / 10% formamide / target (entire

products of 2nd PCR) / 0.05 wt% SDS
```

```
(6 × SSPE: NaCl 900 mM, NaH2PO4·H2O 50 mM, EDTA

6 mM, pH 7.4)
```

3-4. Hybridization condition
$$65°C\ 3min \rightarrow 55°C\ 4hrs \rightarrow wash\ 2 \times SSC\ /\ 0.1\%\ SDS$$

$$at\ 50°C \rightarrow wash\ 2 \times SSC\ at\ 20°C \rightarrow (rinse\ with\ H_2O:$$

$$manual) \rightarrow spin\ dry$$

4. Scanning
After the above described hybridization reaction, the DNA microarray was observed for fluorescence by means of a fluorescence detector for DNA microarrays (GenePix 4000B: manufactured by Axon Instruments). As a result, each of the bacterium species could be detected with a sufficient degree of signaling in a well reproducible manner. The results of fluorescence observation after the hybridization reaction are listed for all the bacterium species in Table 4 below.

[Table 4]

| Probe | Target of detection | Bacterium genome in template | | | | | | | | | |
|---|---|---|---|---|---|---|---|---|---|---|---|
| | | S. aureus | S. epidermidis | E. coli | K. pneumoniae | P. aeruginosa | S. marcescens | S. pneumoniae | H. influenzae | E. cloacae | E. faecalis |
| A | S. aureus | 16000 | 3400 | 60 | 60 | 50 | 60 | 60 | 50 | 60 | 100 |
| B | S. epidermidis | 100 | 16000 | 70 | 60 | 50 | 70 | 60 | 50 | 60 | 50 |
| C | E. coli | 60 | 70 | 19000 | 5000 | 50 | 5000 | 50 | 50 | 3000 | 50 |
| D | K. pneumoniae | 60 | 60 | 300 | 14000 | 50 | 4000 | 50 | 40 | 500 | 50 |
| E | P. aeruginosa | 60 | 50 | 60 | 70 | 8000 | 50 | 60 | 50 | 4000 | 60 |
| F | S. marcescens | 50 | 50 | 80 | 400 | 40 | 9500 | 60 | 60 | 100 | 70 |
| G | S. pneumoniae | 70 | 60 | 70 | 50 | 60 | 50 | 13500 | 60 | 50 | 50 |
| H | H. influenzae | 60 | 70 | 180 | 500 | 100 | 100 | 70 | 3000 | 200 | 50 |
| I | E. cloacae | 50 | 60 | 60 | 12000 | 60 | 50 | 50 | 50 | 7000 | 50 |
| J | E. faecalis | 60 | 50 | 80 | 60 | 50 | 60 | 50 | 50 | 60 | 13500 |

As seen from the above listed results, each of the bacterium species efficiently hybridized with the corresponding probe to show a high luminance value.

While the labeled DNA of each of the bacterium species practically does not hybridize with the probe of any other bacterium species, it may partly give rise to a luminance in some other bacterium species detecting probe. Such a phenomenon is assumed as cross hybridization on the sequences and hence the observed hybridization is a normal hybridization. Thus, a primer set according to the present invention proved to show a highly specific detection effect by analyzing the amplification product.

Example 3 (52 bacterium species)

1. Culture of bacteria

Standard strains of bacteria of the fifty-two species that are main objects of detection for the purpose of the present invention and described in ATCC were obtained by following the predetermined procedures. Some of the strains of bacteria that were not available were obtained from JCM by following the predetermined procedures or from clinical isolates. The obtained strains of bacteria were treated for storage in a proper way and then frozen (-80°C) and stocked. The fifty-two species of bacteria are classified by the types of the culture mediums that were used when they were cultured and listed below.

*S. haemolyticus, S. hominis, S. saprophyticus, P. fluorescens, P. putida, B. cepacia, A. baumannii, B. cereus, B. subtilis, A. calcoaceticus, A. xylosoxidans, S. choleraesuis, M. chelonae, N. asteroides, K. oxytoca, E. aerogenes, H. alvei, S. liquefaciens, P. mirabilis, P. vulgaris, M. morganii, P. rettgeri, A. hydrophila,* (Nutrient Agar culture mediums were used for the above bacteria.) *S. agalactiae, S. mutans, S. pyogenes, S. sanguinis, E. avium, E. faecium, S. maltophilia, C. freundii,*

*A. sobria*

(Soybean Casein Digest Agar culture mediums were used for the above bacteria.)

*V. vulnificus*

(A Marine Agar culture medium was used for the above bacterium.)

*G. vaginalis, B. fragilis, B. thetaiotaomicron, P. acnes, C. difficile, C. perfrigens, E. lenta, F. nucleatum, L. acidophilus, A. prevotii, P. asaccharolyticus, P. asaccharolytica, P. gingivalis,*

(Anaerobic Agar culture medium was used for the above bacteria.)

*C. diphtheriae,*

(A Brain Heat Infusion Agar culture medium was used for the above bacteria.)

*L. pneumophila*

(A Buffered Charcoal Yeast extract Agar culture medium was used for the above bacterium.)

*M. Kansasii, M. intracellulare,*

(2% Ogawa culture mediums were used for the above bacteria.)

*F. necrophorum, P. denticola,*

(Sheep blood agar culture mediums were used for the above bacteria.)

The surfaces of the frozen stocks of bacteria were scraped off to a small extent by a platinum loop and the bacteria obtained by the scraping were applied onto respective culture mediums. The bacteria were cultured at respective temperatures optimum for growing them (30°C to 37°C), while being observed for growth. As a result, all the species were proliferated in about several days. A bacterium solution of an appropriate bacterium concentration was prepared for each species by using a turbidity meter and other tools.

2. Extraction of bacterium genome and human genome

The genome DNA of each of the bacterium species was extracted and purified from the corresponding one of the bacterium culture solutions by means of a nucleic acid purification kit (MORA-EXTRACT manufactured by Kyokuto Pharmaceutical Industrial Co., Ltd.). The collected genome DNA of the bacteria were subjected to agarose electrophoresis and 260/280 nm absorbance measurement by way of an established process to determine the quality (the mixing ratio of low molecular weight nucleic acids, the extent of decomposition) and the collected quantity thereof. In this example, about 5 to 15 $\mu$g of genome DNA originating from the bacterium were collected for each of the extracted fifty-two bacterium species, although the collected quantity varied from species to species. The quality of the collected genome DNA was excellent and neither degradation nor mixed rRNA was observed. The collected genome DNA was diluted by means of TE to prepare a solution containing genome DNA of a million bacteria per 1 $\mu$l.

Meanwhile, genome originating from a human being was extracted from the blood drawn from a healthy person. A nucleic acid purification kit (QIAamp DNA Blood Mini Kit manufactured by QIAGEN) was used to extract a human genome originating from the blood. About 8 $\mu$g of genome DNA were extracted from about 200 $\mu$l of blood. The obtained genome DNA was dissolved in TE to prepare a solution showing a concentration of 200 ng per 1 $\mu$l. The bacterium genome and the human genome were mixed by the same quantities to obtain a model object of examination (specimen) of a genome mixture solution containing 100,000 copies of the bacterium genome and 200 ng of the human genome per 2 $\mu$l.

3. Preparation of primers

Primers were prepared in a manner as described under 3. of Example 1.

4. PCR amplification

A PCR amplification was realized in a manner as described under 4. of Example 1. Note that the template DNA prepared in a manner as described under 2-2 was used for the one shown in Table 1 of Example 1.

5. Electrophoresis

Each of the obtained PCR products was subjected to electrophoresis in order to observe the quantity of amplification obtained by the amplification reaction. A micro-chip type apparatus for electrophoresis "Bioanalyzer" manufactured by Agilent was used for the electrophoresis. 7500 series for DNA was used as the gel kit. Each of the PCR products was applied to a chip of the apparatus by 1.0 μl and subjected to electrophoresis according to the instruction annexed to the apparatus. After the electrophoresis, the synthesized quantity of the PCR product was quantified by means of the software annexed to the apparatus. The chain length of the PCR product to be amplified was about 1,500 bp. Table 5 below shows the PCR products of all the fifty-two bacterium species.

[Table 5]

| Bacterium species | Amplified quantity (concentration) : ng/μl | Bacterium species | Amplified quantity (concentration) : ng/μl |
|---|---|---|---|
| S. haemolyticus | 28.09 | P. rettgeri | 34.4 |
| S. hominis | 22.33 | A. hydrophila | 7.65 |
| S. saprophyticus | 24.08 | S. agalactiae | 26.35 |
| P. fluorescens | 15.2 | S. mutans | 32.59 |
| P. putida | 32.87 | S. pyogenes | 30.11 |
| B. cepa cia | 5.8 | S. sanguinis | 29.89 |
| A. baumannii | 27.96 | E. avium | 19.92 |
| B. cereus | 27.68 | E. faecium | 20.63 |
| B. subtilis | 30.26 | S. maltophilia | 8.2 |
| A. calcoaceticus | 28.1 | C. freundii | 37.03 |
| A. xylosoxidans | 2.1 | A. sobria | 6.27 |
| S. choleraesuis | 19.1 | V. vulnificus | 5.4 |
| M. chelonae | 2.1 | G. vaginalis | 16.17 |
| N. asteroides | 19.87 | B. fragilis | 27.88 |
| K. oxytoca | 31.47 | B. thetaiotaomicron | 21.65 |
| E. aerogenes | 32.94 | P. acnes | 22.22 |
| H. alvei | 12.2 | C. difficile | 30.08 |
| S. liquefaciens | 9.9 | C. perfrigens | 2.2 |
| P. mirabilis | 38.32 | E. lenta | 8.8 |
| P. vulgaris | 37.84 | F. nucleatum | 0.62 |
| M. morganii | 33.66 | L. acidophilus | 5.29 |
| A. prevotii | 11.42 | M. kansasii | 8.7 |
| P. asaccharolyticus | 8.33 | M. intracellulare | 7.5 |
| P. asaccharolytica | 20.15 | F. necrophorum | 3.4 |
| P. gingivalis | 4.6 | P. denticola | 1.58 |
| C. diphtheriae | 3.2 | L. pneumophila | 7.3 |

All the bands show respective lengths that are close to their theoretical chain lengths and no remarkable band was observed with other chain length so that it was confirmed that the genomes originating from the bacteria had been amplified very efficiently. The extent of amplification did not remarkably vary from bacterium species to bacterium species to prove that the primer mix had operated uniformly to all the fifty two bacterium species. While 200 ng of a genome originating from a human being was added per PCR tube in this example, the human genome was not amplified, on the contrary it was proved that a human genome does not inhibit such amplification.

Example 4 (detection by means of DNA microarray)

The results of detection obtained by means of a DNA microarray, using part of the bacteria that are the amplification products of Example 3 will be shown here.

1. Preparation of DNA microarray

A DNA microarray that can identify nine species of bacterium was prepared. The prepared DNA microarray is a DNA microarray designed to identify the bacterium species by analyzing the sequence of 16S rRNA amplified in Example 3 in detail. In other words, the prepared DNA microarray is a DNA microarray where oligonucleotide probes specifically reactive to the respective bacterium species were designed and mounted on a glass carrier as minute spots.

The probes designed respectively for the bacterium species are listed below.

Probe 11: *A prevotii*: 5'CGTTGGAAACGACGAATAATACCCTATGA 3' (sequence No. 24)
Probe 12: *P. asaccharolyticus*: 5'AGTGACACATGTCATAACGATCAAAGTGA 3' (sequence No. 25)
Probe 13: *P. asaccharolytica*: 5' GTGGTGAATAACCCGATGAAAGTCGG 3' (sequence No. 26)
Probe 14: *A. hydrophila:* 5' GGCTGTGACGTTACTCGCAGAAG 3' (sequence No. 27)
Probe 15: *A. sobria*: 5'TAATGCCTGGGGATCTGCCCAG 3' (sequence No. 28)
Probe 16: *B. fragilis: 5'* AATACCCGATAGCATAATGATTCCGCATG 3' (sequence No. 29)
Probe 17: *B. thetaiotaomicron: 5'* CACGTATCCAACCTGCCGATAACTC 3' (sequence No. 30)
Probe 18: *P. acnes:* 5' AAAGTTTCGGCGGTTGGGGATG 3' (sequence No. 31)
Probe 19: *C. difficile*: 5' GCATCTCTTGAATATCAAAGGTGAGCC 3' (sequence No. 32)

The DNA microarray mounted with these oligonucleotide probes was prepared according to the process described in Japanese Patent Application Laid-Open No. 2004-313181.

2. 2nd PCR

A 2nd PCR was conducted as in Example 2-2. The PCR products obtained in Example 3 were employed here.

3. Hybridization

A hybridization reaction was conducted as in Examples 2-3-1 and 2-3-4 by using the DNA microarray prepared in [1. preparation of DNA microarray] and the labeled object of examination prepared in [2. 2nd PCR].

4. Scanning

After the above described hybridization reaction, the DNA microarray was observed for fluorescence by means of a fluorescence detector for DNA microarrays (GenePix 4000B manufactured by Axon Instruments). As a result, each of the bacterium species could be detected with a sufficient degree of signaling in a well reproducible manner. The results of fluorescence observation after the hybridization reaction are listed for all the bacterium species in Table 6 below.

[Table 6]

| Probe | Target of detection | Bacterium genome in template | | | | | | | | |
|---|---|---|---|---|---|---|---|---|---|---|
| | | A. prevotii | P. asaccharolyticus | P. asaccharolytica | A. hydrophila | A. sobria | B. fragilis | B. thetaiotaomicron | P. acnes | C. difficile |
| 11 | A. prevotii | 7499 | 52 | 257 | 49 | 56 | 52 | 48 | 51 | 49 |
| 12 | P. asaccharolyticus | 151 | 2030 | 406 | 49 | 57 | 55 | 45 | 51 | 50 |
| 13 | P. asaccharolytica | 51 | 48 | 1313 | 50 | 56 | 52 | 45 | 52 | 49 |
| 14 | A. hydrophila | 47 | 48 | 330 | 4210 | 55 | 53 | 49 | 52 | 48 |
| 15 | A. sobria | 49 | 47 | 364 | 137 | 6636 | 52 | 49 | 53 | 49 |
| 16 | B. fragilis | 51 | 49 | 320 | 53 | 53 | 7175 | 52 | 52 | 49 |
| 17 | B. thetaiotaomicron | 49 | 47 | 247 | 50 | 53 | 60 | 15238 | 52 | 49 |
| 18 | P. acnes | 48 | 49 | 458 | 50 | 58 | 54 | 47 | 6967 | 48 |
| 19 | C. difficile | 51 | 50 | 157 | 51 | 55 | 53 | 95 | 52 | 4522 |

As seen from the above listed results, each of the bacterium species efficiently hybridized with the corresponding probe to show a high luminance value. While the labeled DNA of each of the bacterium species practically does not hybridize with the probe of any other bacterium species, it may partly give rise to a luminance in some other bacterium species detecting probe. Such a phenomenon is assumed as cross hybridization on the sequences and hence the observed hybridization is a normal hybridization. Thus, a primer set according to the present invention proved to show a highly specific detection effect by analyzing the amplification product.

While a DNA microarray that can identify the nine target bacterium species was used and the results of detection were shown in each of the above-described examples, the same technique can be used for all the forty three remaining bacterium species. A DNA microarray for detecting those bacteria was prepared and employed for hybridization and a high fluorescence luminance was obtained by each of the probes designed for the specific one of the bacterium species. Thus, as a result, each of the bacterium species could be detected with a sufficient degree of signaling in a well reproducible manner.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A primer to be used for PCR amplification of the gene sequence of 16S rRNA of a bacterium, comprising a base sequence selected from the sequences No. 1 through No. 6 listed below:

   (1) GCGGCGTGCCTAATACATGCAAGTCG (sequence No. 1)
   (2) GCGGCAGGCCTAACACATGCAAGTCG (sequence No. 2)
   (3) GCGGCAGGCTTAACACATGCAAGTCG (sequence No. 3)
   (4) ATCCAGCCGCACCTTCCGATACGGC (sequence No. 4)
   (5) ATCCAACCGCAGGTTCCCCTACGG (sequence No. 5)
   (6) ATCCAGCCGCAGGTTCCCCTACGG (sequence No. 6)

2. A primer set for PCR amplification of the gene sequence of 16S rRNA of a bacterium, comprising at least two of the primers (A) through (L) listed below, at least one of which is selected from the primers (A) through (F):

   (A) a primer having GCGGCGTGCCTAATACATGCAAGTCG (sequence No. 1),
   (B) a primer having GCGGCAGGCCTAACACATGCAAGTCG (sequence No. 2),
   (C) a primer having GCGGCAGGCTTAACACATGCAAGTCG (sequence No. 3),
   (D) a primer having ATCCAGCCGCACCTTCCGATACGGC (sequence No. 4),
   (E) a primer having ATCCAACCGCAGGTTCCCCTACGG (sequence No. 5),
   (F) a primer having ATCCAGCCGCAGGTTCCCCTACGG (sequence No. 6),
   (G) a primer having GCGGCGTGCCTAATACATGCAAG (sequence No. 7),
   (H) a primer having GCGGCAGGCCTAACACATGCAAG (sequence No. 8),
   (I) a primer having GCGGCAGGCTTAACACATGCAAG (sequence No. 9),
   (J) a primer having ATCCAGCCGCACCTTCCGATAC (sequence No. 10),
   (K) a primer having ATCCAACCGCAGGTTCCCCTAC (sequence No. 11) and
   (L) a primer having ATCCAGCCGCAGGTTCCCCTAC (sequence No. 12).

3. The primer set according to claim 2 containing at least the primers (D) through (I).

4. The primer set according to claim 2 containing at least the primers (D), (F), (G), (H), (I) and (K).

5. A reagent kit for identifying a bacterium, the kit containing a primer according to claim 1 or the primer set according to any of claims 2 through 4.

6. A bacterium detection method for executing a DNA amplification process, using the primer according to claim 1 or the primer set according to any of claims 2 through 4 and detecting a bacterium DNA by means of a DNA probe.

7. The primer according to claim 1 or the primer set according to any of claims 2 through 4, wherein the bacterium is selected from a group of *Genus Staphylococcus, Genus Streptococcus, Genus Enterococcus, Genus Pseudomonas, Genus Enterobacter, Genus Haemophilus, Genus Serratia, Genus Klebsiella, Genus Escherichia, Genus Burkholderia, Genus Stenotrophomonas, Genus Acinetobacter, Genus Achromobacter, Genus Vibrio, Genus Salmonella, Genus Citrobacter, Genus Hafnia, Genus Proteus, Genus Morganella, Genus Providencia*, *Genes Aeromonas, Genus Gardnerella, Genus Corynebacterium, Genus Legionella, Genus Bacillus, Genus Mycobacterium, Genus Nocardia, Genus Bacteroides, Genus Clostridium, Genus Eggerthella, Genus Fusobacterium, Genus Lactobacillus, Genus Anaerococcus, Genus Peptoniphilus, Genus Porphyromonas, Genus Prevotella and Genus Propionibacterium.*

EP 1 927 664 A1

**European Patent Office**

# EUROPEAN SEARCH REPORT

Application Number

EP 07 02 2954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2006 129810 A (CANON KK) 25 May 2006 (2006-05-25) SEQ ID NOs 195, 196, 197, 204, 205, 206 * abstract * * tables 2,4,5 * ----- | 1-7 | INV. C12Q1/68 |
| X | US 2004/241643 A1 (YAMAMOTO NOBUKO [JP] ET AL) 2 December 2004 (2004-12-02) SEq ID NOs: 107, 108,109,110,111 * abstract * * examples 4,5; table 11 * ----- | 1-7 | |

TECHNICAL FIELDS SEARCHED (IPC)

C12Q

The present search report has been drawn up for all claims

| Place of search | Date of completion of the search | Examiner |
|---|---|---|
| Munich | 27 March 2008 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

24

## ANNEX TO THE EUROPEAN SEARCH REPORT
## ON EUROPEAN PATENT APPLICATION NO.

EP 07 02 2954

This annex lists the patent family members relating to the patent documents cited in  the above-mentioned European search report.
The members are as contained in the European Patent Office EDP file on
The European Patent Office is in no way liable for these particulars which are merely given for the purpose of information.

27-03-2008

| Patent document cited in search report | | Publication date | Patent family member(s) | | Publication date |
|---|---|---|---|---|---|
| JP 2006129810 | A | 25-05-2006 | NONE | | |
| US 2004241643 | A1 | 02-12-2004 | CN | 1539994 A | 27-10-2004 |
| | | | EP | 1464710 A2 | 06-10-2004 |
| | | | JP | 2004313181 A | 11-11-2004 |
| | | | KR | 20040086612 A | 11-10-2004 |
| | | | KR | 20060106793 A | 12-10-2006 |
| | | | KR | 20070057732 A | 07-06-2007 |
| | | | KR | 20070112744 A | 27-11-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001299396 A
- JP H06133798 A
- JP H10394896 A
- JP H10304897 A
- JP 2004313181 A

SEQUENCE LISTING

<110> Canon Kabushiki Kaisha

<120> PRIMER FOR BACTERIUM GENOME AMPLIFICATION REACTION

<130> 10048391EP01

<150> JP2006-319976
<151> 2006-11-28

<160> 32

<170> PatentIn version 3.3

<210> 1
<211> 26
<212> DNA
<213> Artificial

<220>
<223> Primer for amplificatio of microoranism genome

<400> 1
gcggcgtgcc taatacatgc aagtcg          26


<210> 2
<211> 26
<212> DNA
<213> Artificial

<220>
<223> Primer for amplificatio of microoranism genome

<400> 2
gcggcaggcc taacacatgc aagtcg          26


<210> 3
<211> 26
<212> DNA
<213> Artificial

<220>
<223> Primer for amplificatio of microoranism genome

<400> 3
gcggcaggct taacacatgc aagtcg          26


<210> 4
<211> 25
<212> DNA
<213> Artificial

<220>
<223> Primer for amplificatio of microoranism genome

<400> 4
atccagccgc accttccgat acggc          25

```
<210>  5
<211>  24
<212>  DNA
<213>  Artificial

<220>
<223>  Primer for amplificatio of microoranism genome

<400>  5
atccaaccgc aggttcccct acgg                                            24


<210>  6
<211>  24
<212>  DNA
<213>  Artificial

<220>
<223>  Primer for amplificatio of microoranism genome

<400>  6
atccagccgc aggttcccct acgg                                            24


<210>  7
<211>  23
<212>  DNA
<213>  Artificial

<220>
<223>  Primer for amplificatio of microoranism genome

<400>  7
gcggcgtgcc taatacatgc aag                                             23


<210>  8
<211>  23
<212>  DNA
<213>  Artificial

<220>
<223>  Primer for amplificatio of microoranism genome

<400>  8
gcggcaggcc taacacatgc aag                                             23


<210>  9
<211>  23
<212>  DNA
<213>  Artificial

<220>
<223>  Primer for amplificatio of microoranism genome

<400>  9
gcggcaggct taacacatgc aag                                             23


<210>  10
<211>  22
<212>  DNA
```

<210> Artificial

<220>
<223> Primer for amplificatio of microoranism genome

<400> 10
atccagccgc accttccgat ac                                    22


<210> 11
<211> 22
<212> DNA
<213> Artificial

<220>
<223> Primer for amplificatio of microoranism genome

<400> 11
atccaaccgc aggttcccct ac                                    22


<210> 12
<211> 22
<212> DNA
<213> Artificial

<220>
<223> Primer for amplificatio of microoranism genome

<400> 12
atccagccgc aggttcccct ac                                    22


<210> 13
<211> 25
<212> DNA
<213> Artificial

<220>
<223> Probe for detection of microorganism genome

<400> 13
taacctttta ggagctagcc gtcga                                 25


<210> 14
<211> 24
<212> DNA
<213> Artificial

<220>
<223> Probe for detection of microorganism genome

<400> 14
agtaaccatt tggagctagc cgtc                                  24


<210> 15
<211> 25
<212> DNA
<213> Artificial

<220>

<223> Probe for detection of microorganism genome

<400> 15
cggacctcat aaagtgcgtc gtagt                                    25


<210> 16
<211> 22
<212> DNA
<213> Artificial

<220>
<223> Probe for detection of microorganism genome

<400> 16
cctttgttgc cagcggttag gc                                       22


<210> 17
<211> 25
<212> DNA
<213> Artificial

<220>
<223> Probe for detection of microorganism genome

<400> 17
tggccttgac atgctgagaa ctttc                                    25


<210> 18
<211> 27
<212> DNA
<213> Artificial

<220>
<223> Probe for detection of microorganism genome

<400> 18
gaaactggca agctagagtc tcgtaga                                  27


<210> 19
<211> 26
<212> DNA
<213> Artificial

<220>
<223> Probe for detection of microorganism genome

<400> 19
gacggcaagc taatctctta aagcca                                   26


<210> 20
<211> 26
<212> DNA
<213> Artificial

<220>
<223> Probe for detection of microorganism genome

<400> 20

ggcgtttacc acggtatgat tcatga                                    26

<210> 21
<211> 25
<212> DNA
<213> Artificial

<220>
<223> Probe for detection of microorganism genome

<400> 21
attcgaaact ggcaggctag agtct                                     25

<210> 22
<211> 29
<212> DNA
<213> Artificial

<220>
<223> Probe for detection of microorganism genome

<400> 22
cgaggtcatg caaatctctt aaagcttct                                 29

<210> 23
<211> 23
<212> DNA
<213> Artificial

<220>
<223> Primer for amplification of microoganism genome

<400> 23
taccttgtta cgacttcacc cca                                       23

<210> 24
<211> 29
<212> DNA
<213> Artificial

<220>
<223> Probe for detection of microorganism genome

<400> 24
cgttggaaac gacgaataat accctatga                                 29

<210> 25
<211> 29
<212> DNA
<213> Artificial

<220>
<223> Probe for detection of microorganism

<400> 25
agtgacacat gtcataacga tcaaagtga                                 29

<210> 26
<211> 26
<212> DNA
<213> Artificial

<220>
<223> Probe for detection of microorganism

<400> 26
gtggtgaata acccgatgaa agtcgg          26


<210> 27
<211> 23
<212> DNA
<213> Artificial

<220>
<223> Probe for detection of microorganism

<400> 27
ggctgtgacg ttactcgcag aag          23


<210> 28
<211> 22
<212> DNA
<213> Artificial

<220>
<223> Probe for detection of microorganism

<400> 28
taatgcctgg ggatctgccc ag          22


<210> 29
<211> 29
<212> DNA
<213> Artificial

<220>
<223> Probe for detection of microorganism

<400> 29
aatacccgat agcataatga ttccgcatg          29


<210> 30
<211> 25
<212> DNA
<213> Artificial

<220>
<223> Probe for detection of microorganism

<400> 30
cacgtatcca acctgccgat aactc          25


<210> 31
<211> 22
<212> DNA

```
<213>  Artificial

<220>
<223>  Probe for detection of microorganism

<400>  31
aaagtttcgg cggttgggga tg                                          22


<210>  32
<211>  27
<212>  DNA
<213>  Artificial

<220>
<223>  Probe for detection of microorganism

<400>  32
gcatctcttg aatatcaaag gtgagcc                                     27
```